# EUROPEAN PATENT APPLICATION

(11) **EP 4 431 051 A1**
(43) Date of publication of application: **18.09.2024**
(21) Application number: 22891853.8
(22) Date of filing: 01.11.2022
(51) Int. Cl.: A61B 34/37, A61B 34/00, B25J 19/00

(54) **WRIST OF MASTER MANIPULATOR, AND MASTER OPERATING APPARATUS AND SURGICAL ROBOT**

(30) Priority: 11.11.2021 CN 202111345890
(71) Applicant: Shenzhen Edge Medical Co., Ltd., Shenzhen, Guangdong 518000 (CN)
(72) Inventor: LIU, Fang, Shenzhen, Guangdong 518000 (CN)
(74) Representative: Biallo, Dario
(86) International application number: PCT/CN2022/129011
(87) International publication number: WO 2023/083057

(57) **Abstract**

A master-hand wrist (23), a master operating apparatus (20) and a surgical robot. The master-hand wrist (23) comprises: a handle (234); a first rod (231) rotatably connected to the handle (234) about a first rotation axis (J1) via a first rotation joint (235); a second rod (232) rotatably connected to the first rod (231) about a second rotation axis (J2) via a second rotation joint (236); and a third rod (233) rotatably connected to the second rod (232) about a third rotation axis (J3) via a third rotation joint (237), wherein the third rotation joint (237) is provided with a torque compensation mechanism about the third rotation axis (J3) to compensate for torque generated by the handle (234), the first rod (231) and the second rod (232) about the third rotation axis(J3). The master-hand wrist (23), the master operating apparatus (20), and the surgical robot provided by the present disclosure utilize torque compensation to achieve the effect of driving the rotation joint with less driving force, which effectively reduces the inertia effect of the distal end and increases force transparency.

## Description

This application claims the priority of Chinese Patent Application No. 202111345890.6, filed on Nov. 11, 2021, and titled "master-hand wrist, master operating apparatus, and surgical robot", the entirety of which is incorporated by reference herein.

### TECHNICAL FIELD

The subject matter herein generally relates to medical instruments, in particular to a master-hand wrist, a master operating apparatus comprising the master-hand wrist, and a surgical robot comprising the master operating apparatus.

### BACKGROUND

Minimally invasive surgery refers to a surgical method that uses modern medical instruments such as laparoscopy and thoracoscopy and related equipment to perform surgery inside the human cavity. Compared with traditional surgical methods, minimally invasive surgery has the advantages of less trauma, less pain, and faster recovery.

With the advancement of science and technology, technology of minimally invasive surgical robot is gradually maturing and widely used. A minimally invasive surgical robot usually includes a master operating platform and a slave operating apparatus. The master operating platform is configured to send control commands to the slave operating apparatus based on the doctor's operation to control the slave operating apparatus. The slave operating apparatus is configured to perform corresponding surgical operation in response to the control commands from the master operating platform. A surgical instrument is connected to a driving device of the slave operating apparatus to perform surgical operations, and a terminal instrument of the surgical instrument includes an end effector for performing surgical operations and a joint connected to the end effector that can move in multiple degrees of freedom.

In surgery using the surgical robot, the doctor needs to operate the Master Hand (MH) for a long time. The closer to the distal end of the gimbal mechanism (Gimbal) of the traditional master hand, the greater the load, the greater motor power required, resulting in low operating comfort of the doctor.

### SUMMARY

Based on the aforementioned technical problems, it is necessary to provide a master-hand wrist, a master operating apparatus comprising the master-hand wrist, and a surgical robot comprising the master operating apparatus aiming at solving the aforementioned technical problems.

The first aspect of the embodiment of the present disclosure provides a master-hand wrist. The master-hand wrist includes a handle; a first rod rotatably connected to the handle about a first rotation axis via a first rotation joint; a second rod rotatably connected to the first rod about a second rotation axis via a second rotation joint; and a third rod rotatably connected to the second rod about a third rotation axis via a third rotation joint; wherein the third rotation joint is provided with a torque compensation mechanism about the third rotation axis to compensate for the torque generated by the handle, the first rod, and the second rod about the third rotation axis.

In an embodiment, the third rotation joint comprises a third rotation shaft arranged along the third rotation axis and a driving element mounted on the third rotation shaft, one end of the third rotation shaft is fixedly connected to the second rod, the other end of the third rotation shaft is rotatably connected to the third rod, and the driving element provides power for the third rotation shaft to rotate about the third rotation axis.

In an embodiment, the driving element comprises a motor rotor and a motor stator, the motor rotor is fixed on the third rotation shaft, and the motor stator is coaxially arranged outside the motor rotor.

In an embodiment, the torque compensation mechanism comprises a movable pulley eccentrically mounted on the third rotation shaft, a first fixed pulley concentrically mounted on the third rotation shaft, a second fixed pulley concentrically mounted on a fixed shaft parallel to the third rotation shaft, an elastic element, and a rope having one end fixed relative to the third rod and the other end wound around the movable pulley, the first fixed pulley, the second fixed pulley and connected to one end of the elastic element sequentially, and the other end of the elastic element is fixed on the third rod.

In an embodiment, the fixed shaft is mounted inside the third rod and above the third rotation shaft, and diameters of sliding portions of the movable pulley, the first fixed pulley, and the second fixed pulley are equal.

In an embodiment, the torque compensation mechanism comprises a guide element fixed on the third rod and disposed on a portion of the rope between the second fixed pulley and the elastic element, and the rope is wound around the guide element so that a portion of the rope between the guide element and the elastic element is in a vertical direction.

In an embodiment, the elastic element is a tensile elastic element, and an initial length of the elastic element is determined by achieving torque balance of the third rotation shaft at an initial position; a stiffness coefficient of the elastic element is determined based on a relationship between a rotation angle of the third rotation shaft relative to the third rod and magnitude of required compensation torque.

In an embodiment, the first rotation joint comprises a first rotation shaft arranged along the first rotation axis and a first driving element mounted on the first rotation shaft, one end of the first rotation shaft is fixedly connected to the handle, the other end of the first rotation shaft is rotatably connected to the first rod, and the first driving element provides power for the first rotation shaft to rotate about the first rotation axis; the second rotation joint comprises a second rotation shaft arranged along the second rotation axis and a second driving element mounted on the second rotation shaft, one end of the second rotation shaft is fixedly connected to the first rod, the other end of the second rotation shaft is rotatably connected to the second rod, and the second driving element provides power for the second rotation shaft to rotate about the second rotation axis. In an embodiment, at least one of the first rotation joint and the second rotation joint is provided with a torque compensation mechanism.

The second aspect of the embodiment of the present disclosure provides a master-hand wrist. The master-hand wrist includes a handle; a first rod rotatably connected to the handle about a first rotation axis via a first rotation joint; a second rod rotatably connected to the first rod about a second rotation axis via a second rotation joint; a third rod rotatably connected to the second rod about a third rotation axis via a third rotation joint; and at least one torque compensation mechanism to compensate for torque generated by at least one of the handle, the first rod, the second rod, and the third rod about the first, the second, or the third rotation axis.

In an embodiment, the at least one torque compensation mechanism is one torque compensation mechanism connected to the third rod for compensating torque generated by the handle, the first rod, and the second rod about the third rotation axis.

In an embodiment, the at least one torque compensation mechanism are a plurality of torque compensation mechanisms.

The third aspect of the embodiment of the present disclosure provides a master operating apparatus. The master operating apparatus comprises a master operating platform, a master-hand arm, and the aforementioned master-hand wrist, wherein the other end of the third rod is mounted on the master-hand arm via a fourth rotation joint, and the master operating platform processes input signals from the arm and the master-hand wrist.

The fourth aspect of the embodiment of the present disclosure provides a surgical robot. The surgical robot comprises a slave operating apparatus and the aforementioned master operating apparatus, wherein the slave operating apparatus performs corresponding operation in response to command from the master operating apparatus.

The master-hand wrist, the master operating apparatus, and the surgical robot provided by the present disclosure at least offer the following advantages.

The master-hand wrist, the master operating apparatus, and the surgical robot provided by the present disclosure utilize torque compensation to achieve the effect of driving the rotation joint with less driving force, which effectively reduces the inertia effect of the distal end and increases force transparency.

### BRIEF DESCRIPTION OF THE DRAWINGS

FIG. 1 is a schematic view of a surgical robot according to an embodiment of the present disclosure;
FIG. 2 is a perspective view of a master-hand wrist according to an embodiment of the present disclosure;
FIG. 3 is a side view of the master-hand wrist of FIG. 2;
FIG. 4 is a top view of the master-hand wrist of FIG. 2;
FIG. 5 is a cross-section view of the master-hand wrist of FIG. 4, taken along line A-A thereof;
FIG. 6 is an exploded view of the master-hand wrist of FIG. 2 from a different aspect;
FIG. 7 is an exploded view of the master-hand wrist of FIG. 6;
FIG. 8 is an enlarged assembled view of the torque compensation mechanism of FIG. 7;
FIG. 9 is a side view of the master-hand wrist of FIG. 6 with the second housing cut away to illustrate the torque compensation mechanism in a first state;
FIG. 10 is a side view of the master-hand wrist of FIG. 6 with the second housing cut away to illustrate the torque compensation mechanism in a second state;
FIG. 11 is a side view of the master-hand wrist of FIG. 6 with the second housing cut away to illustrate the torque compensation mechanism in a third state.

Reference signs in the drawings are listed as follows:
slave operating apparatus 10 (comprising mechanical arms 11, instruments 12, instrument driving devices 13);
master operating apparatus 20 (comprising master operating platform 21, master-hand arm 22, master-hand wrist 23; first rod 231, second rod 232, third rod 233, handle 234, first rotation joint 235, second rotation joint 236, third rotation joint 237, fourth rotation joint 238, first rotation axis J1, second rotation axis J2, third rotation axis J3, first housing 2331, second housing 2332, third rotation shaft 2371, motor rotor 2372, motor stator 2373, movable pulley 2374, first fixed pulley 2375, second fixed pulley 2376, guide element 2377, elastic element 2378, rope 2379).

### DETAILED DESCRIPTION

The present disclosure will be described clearly and thoroughly herein by accompanying with appended figures of some embodiments. Apparently, the embodiments are only component of the present disclosure, and are not the whole disclosure. For the person of ordinary skill in the art, other embodiments may be obtained based on the provided embodiments without any creative work, and the other embodiments are also covered by the present disclosure. The preferred embodiments of the present disclosure are illustrated in the accompanying drawings. However, it should be understood that the present disclosure can be implemented in many different forms and is not limited to the embodiments described herein. On the contrary, these embodiments are provided to enable a more thorough and comprehensive understanding of the present disclosure.

It should be noted that when an element is referred to as being "disposed on" or "provided on" another element, it can be directly on the other element or intervening elements may also be present. When an element is considered to be "connected" to another element, it can be directly connected to the other element or intervening elements may also be present. Similarly, when an element is considered to be "coupled" to another element, it can be directly coupled to the other element or intervening elements may also be present. The term " engaged " in the present disclosure refers to a coupling of two elements with power transmission. The terms "vertical", "horizontal", "left", "right", "above", "below", and similar expressions in the present disclosure are used for illustrative purposes only and do not represent exclusive embodiments. It should be understood that these space-related terms are intended to encompass different orientations of the device in use or in operation in addition to those depicted in the accompanying drawings, e.g., different orientations of the device in use or in operation, e.g., different orientations of the device in use or in operation. It should be understood that these space-related terms are intended to cover different orientations of the device in use or in operation in addition to those depicted in the accompanying drawings. For instance, if the apparatus is turned over in the accompanying drawings, elements or features described as being "below" or "under" other elements or features will be oriented as being "above" other elements or features. Thus, the example term "below" may include both above and below orientations.

The terms "distal" and "proximal" are used herein as orientation terms and are commonly used in the field of interventional medical instrument, wherein "distal" refers to the end that is away from the operator during the surgical procedure and "proximal" refers to the end that is close to the operator during the surgical procedure. The term "plurality" as used herein includes two or more. The term " coupled" used herein can be broadly understood as any event in which two or more objects are connected in a manner. This manner allows absolutely coupled objects to operate with each other in such a way that there is no relative movement between the objects in at least one direction. For instance, a protrusion and a recess are coupled in such a way that they can move relative to each other in the radial direction but not in the axial direction.

The term "instrument" is used herein to describe medical instrument configured to be inserted into a patient's body and perform a surgical or diagnostic procedure. The instrument includes an end effector, and the end effector may be a surgical tool used for performing a surgical procedure, such as an electrocautery cauterizer, forceps, anastomose, clipper, imaging device (e.g., endoscope or ultrasound probe), and the like. Some of the instruments used in embodiments of the present disclosure further include articulating components (e.g., joint assemblies) provided for the end effector such that the position and orientation of the end effector can be changed by manipulation in one or more mechanical degrees of freedom relative to the axis of the instrument. Furthermore, the end effector includes a functional mechanical degree of freedom, such as open and closed jaws. The instrument may also include stored information that can be updated by the surgical system. One-way or two-way communication between the instrument and one or more system components may be provided via the storage system.

Unless otherwise defined, all technical and scientific terms used herein have the same meaning as commonly understood by one of ordinary skill in the art to which this application belongs. The terms used in the specification of the present disclosure are for the purpose of describing specific embodiments only and are not intended to limit the application. The term "and/or" as used herein includes any and all combinations of one or more of the associated listed items.

In the present disclosure, the term "instrument" is used to describe a medical device designed for insertion into a patient's body to perform surgical or diagnostic procedures. This instrument includes an end effector, which can be a surgical tool related to surgical operations, such as an electrocautery device, grasper, stapler, cutter, imaging device (e.g., endoscope or ultrasonic probe), and the like. Some instruments used in embodiments of this application further include articulated components (e.g., joint assemblies) that provide the end effector with the ability to manipulate its position and orientation with one or more mechanical degrees of freedom relative to the instrument shaft. Furthermore, the end effector incorporates additional functional mechanical degrees of freedom, such as opening and closing jaws. The instrument may also comprise stored information that can be updated by the surgical system, thereby enabling unidirectional or bidirectional communication between the instrument and one or more system components.

Unless otherwise defined, all technical and scientific terms used herein have the same meaning as commonly understood by one of ordinary skill in the art to which this application belongs. The terminology employed herein is solely for the purpose of describing specific embodiments and is not intended to limit the scope of this application. The terms "and/or" and "or/and" as used herein encompass any and all combinations of one or more of the associated listed items.

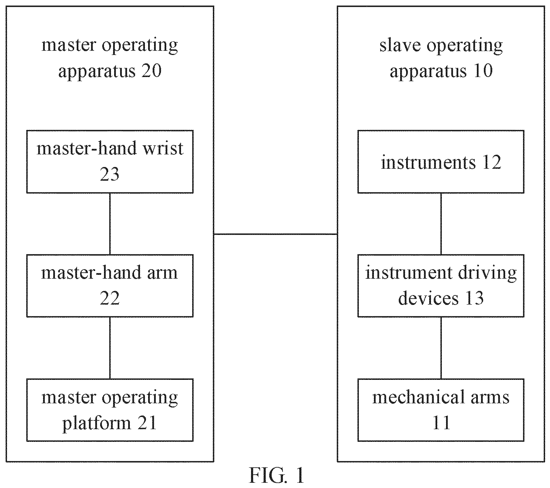

Referring to FIG. 1, a surgical robot of an embodiment according to the present disclosure comprises a slave operating apparatus 10 and a master operating apparatus 20. The slave operating apparatus 10 is located on the patient's side and configured to perform surgical procedure. The slave operating apparatus 10 includes multiple mechanical arms 11 and instruments 12 mounted on the mechanical arms 11. The instruments 12 can comprise electrocautery device, grasper, stapler, cutter, etc. used for surgical operations, camera for capturing images, and other surgical instruments. Multiple instruments 12 are driven by corresponding instrument driving devices 13 respectively to be inserted into the patient's body through different incisions. The mechanical arms 11 are configured to be supported by a pillar via multiple upper arms. In some other embodiments, the mechanical arms 11 of the slave operating apparatus 10 may also be mounted on a wall or ceiling.

The surgical robot typically further includes an imaging system (not shown in drawings) that allows the operator to observe the surgical area from exterior of the patient's body. The imaging system usually comprises one or more devices configured to capture images (such as an instrument 12 with the image capture capability) and one or more video display devices configured to display the captured images. Typically, the instrument 12 with the image capture capability includes optics comprising one or more imaging sensors (such as CCD or CMOS) configured to capture images inside the patient's body. The one or more imaging sensors can be arranged at the distal end of the instrument 12 with the image capture capability, and the signals generated by the one or more sensors can be transmitted along cables or wirelessly to be processed and displayed by the video display device.

The master operating apparatus 20 is located on the operator's side and configured to transmit control commands to the slave operating apparatus 10 based on the operator's operation and display images from the slave operating apparatus 10. The operator can observe the three-dimensional image inside the patient's body provided by the imaging system through the master operating apparatus 20. The operator can control the slave operating apparatus 10 to perform corresponding operation (such as performing surgery or capturing images inside the patient's body) with an immersive feeling by operating the master operating apparatus 20 and observing the three-dimensional image inside the patient's body.

The master operating apparatus 20 includes a master operating platform 21, a master-hand arm 22, and a master-hand wrist 23. The master operating platform 21 may include a display device, a control signal processing system, and an observation device. The display device is configured to display image captured by the aforementioned imaging system, and the observation device is configured to observe the image displayed by the display device. The observation device can be omitted if necessary, and the image can be observed directly. The operator controls the movement of the slave operating apparatus 10 by manipulating the master-hand arm 22 and master-hand wrist 23. The control signal processing system of the master operating platform 21 processes the input signals from the master-hand arm 22 and master-hand wrist 23 and sends control commands to the slave operating apparatus 10. The slave operating apparatus 10 is configured to perform corresponding operations in response to the control commands from the master operating platform 21.

Specifically, a pose mapping between the master-hand arm 22, master-hand wrist 23, and the mechanical arms 11 and instruments of the slave operating apparatus 10 is established through the master operating apparatus 20. The mapping can be corresponded to a positional relationship, which can be distance proportionality, distance trend correspondence, etc.. Alternatively, the mapping can be corresponded to a motion relationship, which can be motion posture correspondence, motion trend correspondence, etc.. Thus, the operator can control the instruments 12 to perform corresponding actions (such as pitching, yawing, rolling, clamping, etc.) by operating the master-hand arm 22 and master-hand wrist 23. The master-hand arm 22 and master-hand wrist 23 can be directly arranged on the master operating platform 21. In other embodiments, the master-hand arm 22 and master-hand wrist 23 can be arranged separately from the master operating platform 21.

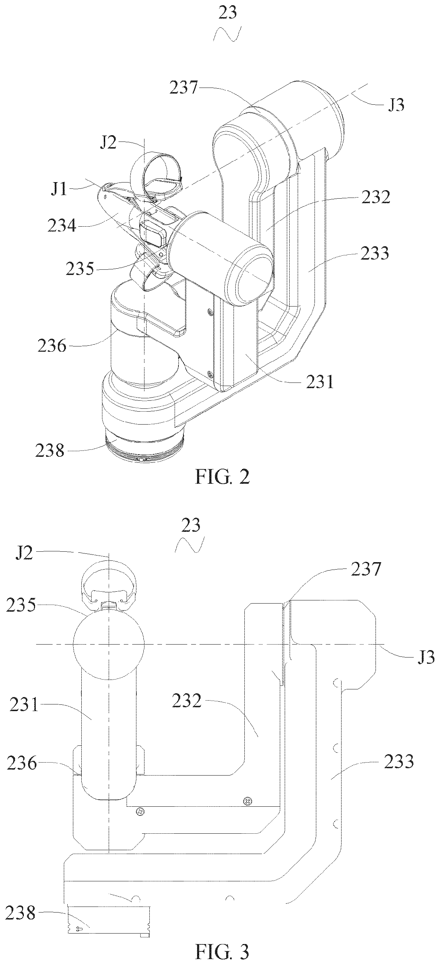

Referring to FIGs. 2 to 4. The master-hand wrist 23 has multiple degrees of freedom. Typically, the master-hand wrist 23 has at least three degrees of freedom. The master-hand wrist 23 comprises a first rod 231, a second rod 232, a third rod 233, a handle 234, a first rotation joint 235, a second rotation joint 236, a third rotation joint 237, and a fourth rotation joint 238. The handle 234 is mounted to one end of the first rod 231 via the first rotation joint 235, and the other end of the first rod 231 is mounted to one end of the second rod 232 via the second rotation joint 236; the other end of the second rod 232 is mounted to one end of the third rod 233 via the third rotation joint 237; the other end of the third rod 233 is mounted to the master-hand arm 22 via the fourth rotation joint 238.

The handle 234 is rotatably connected to the first rod 231 via the first rotation joint 235, enabling the handle 234 to rotate about a first rotation axis J1 of the first rotation joint 235. The first rod 231 is rotatably connected to the second rod 232 via the second rotation joint 236, enabling the first rod 231 to rotate about a second rotation axis J2 of the second rotation joint 236. The second rod 232 is rotatably connected to the third rod 233 via the third rotation joint 237, enabling the second rod 232 to rotate about a third rotation axis J3 of the third rotation joint 237. The third rod 233 is rotatably connected to the master-hand arm 22 via the fourth rotation joint 238, enabling the third rod 233 to rotate about the rotation axis of the fourth rotation joint 238.

In the illustrated embodiment, the rotation axes of the first rotation joint 235, the second rotation joint 236, and the third rotation joint 237-namely, the first rotation axis J1, the second rotation axis J2, and the third rotation axis J3-intersect at one point. The design of master-hand wrist 23 with multiple axes intersecting at one point can decouple orientation and position of the entire master hand and facilitate kinematic calculations.

In the illustrated embodiment, the first rod 231, the second rod 232, and the third rod 233 are L-shaped rods, with each rod having two ends connected perpendicularly to each other. Both ends of each rod are connected to the rotation joints.

In the illustrated embodiment, the gripper (not shown in drawings) on the handle 234 can perform movements in multiple degrees of freedom through the handle 234, multiple rods (first rod 231, second rod 232, third rod 233), and multiple rotation joints (first rotation joint 235, second rotation joint 236, third rotation joint 237, fourth rotation joint 238). In other embodiments, the number of rods and rotation joints can be configured according to the number of motion degree(s) of freedom actual required by the gripper.

The master-hand arm 22 is provided with a mounting end and a connecting end, wherein the mounting end is capable of being fixedly connected to a supporting base of the master operating apparatus 20. The master-hand arm 22 has at least one degree of freedom of movement. The master-hand wrist 23 is movably arranged on the connecting end of the master-hand arm 22 via the fourth rotation joint 238, allowing the operator to perform corresponding operation, such as rotating or gripping. The movement of the gripper 210 relative to the handle 234 in the open-closed degree of freedom can control the opening and closing actions (e.g., gripping or shearing) of the end effector by being mapped to the instrument 12. The rotation of at least one of the gripper and the handle 234 about the first rotation axis J1 of the first rotation joint 235 in the rotating degree of freedom can control the rotating action of the end effector of the instrument 12 by being mapped to the instrument 12. In the aforementioned master operating apparatus 20, the master-hand wrist 23 is mounted on the master-hand arm 22, facilitating the operator's manipulation based on actual working conditions.

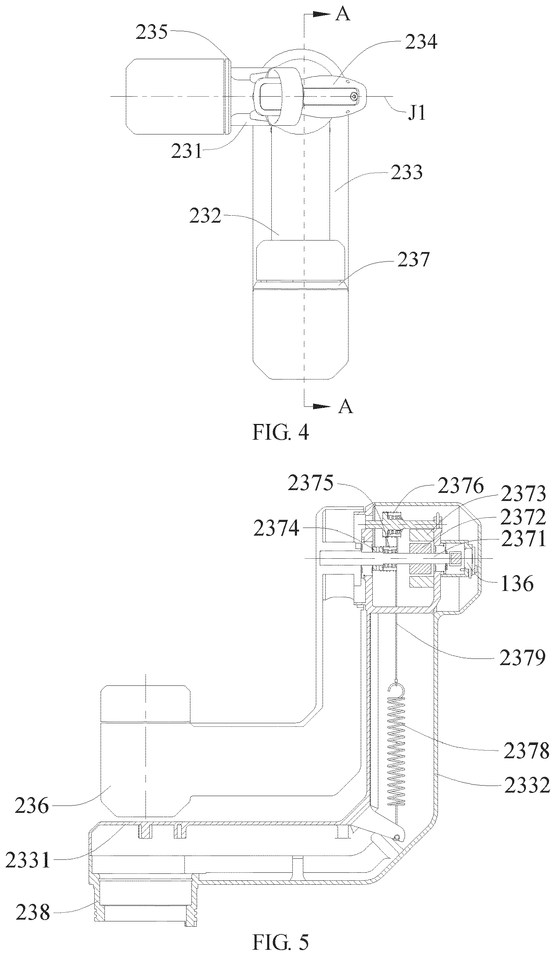

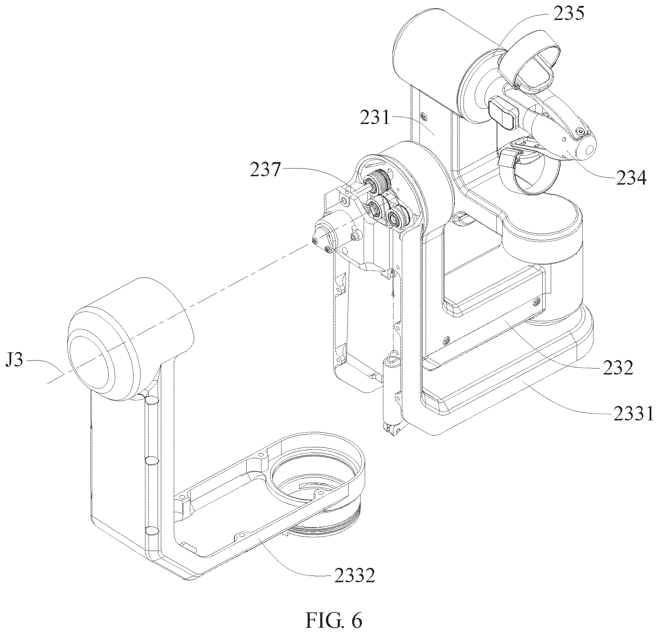

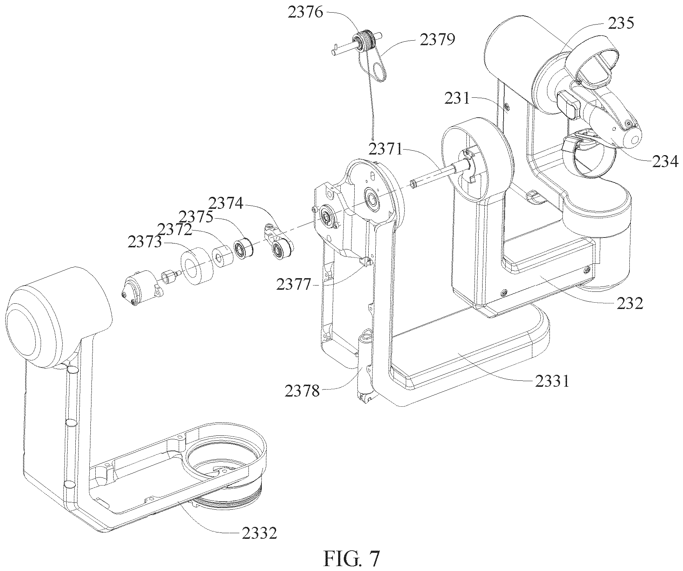

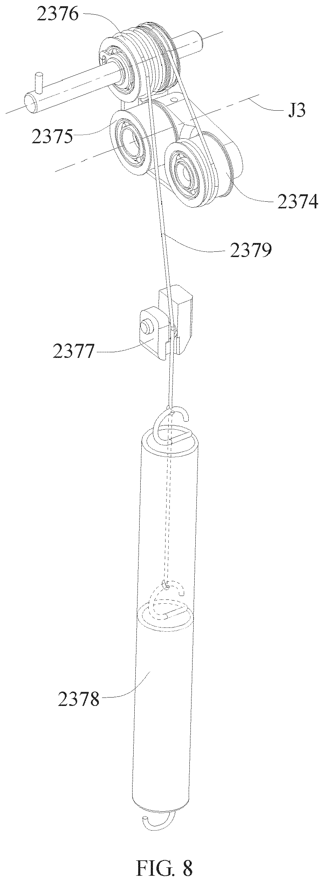

Referring to FIGs. 5 to 8, in an embodiment of the present disclosure, the third rotation joint 237 comprises a third rotation shaft 2371, a motor rotor 2372, and a motor stator 2373. The third rotation shaft 2371 is arranged along the third rotation axis J3, with one end fixedly connected to the second rod 232 and the other end rotatably mounted on the third rod 233 via bearing etc.. The motor rotor 2372 is disposed on the third rotation shaft 2371, and the motor stator 2373 is engaged with the motor rotor 2372. The motor stator 2373 provides power for the motor rotor 2372. The motor rotor 2372 drives the third rotation shaft 2371 to rotate about the third rotation axis J3 by the power from the motor stator 2372.

In the illustrated embodiment, the third rotation shaft 2371 is driven by a motor as the driving element, and the motor's power is transmitted to the third rotation shaft 2371 through the motor rotor 2372 and motor stator 2373. It is understood that in other embodiments, the third rotation shaft 2371 can also adopt other power source and transmission method. Additionally, the motor can adopt a hollow-cup-motor directly driving method to avoid issues such as backlash and transmission delay caused by the reducer.

Meanwhile, torque compensation mechanism(s) can be provide in at least one of the rotation joint of the master-hand arm 22, the rotation joint of the master-hand wrist 23, and the rotation joint between the master-hand arm 22 and the master-hand wrist 23 (fourth rotation joint 238), which can effectively compensate for/balance the joint gravity torque caused by the weight of at least one of the master-hand arm 22 and master-hand wrist 23 at the rotation joint(s), thereby alleviating or avoiding fatigue caused by long-term operation of the operator, and improving surgical efficiency and precision.

Optionally, the movement of at least one of the master-hand arm 22 and the master-hand wrist 23 can be translational, rotation, or other types of movement. Correspondingly, the torque compensation mechanism adapts according to the movement type of at least one of the master-hand arm 22 and the master-hand wrist 23. The following example only illustrates the rotation of the internal joints of the master-hand wrist 23. It is understood that when at least one of the master-hand arm 22 and the master-hand wrist 23 performs other types of movement, the torque compensation mechanism can be reasonably modified based on the form in the following embodiment.

The torque compensation mechanism comprises a movable pulley 2374, a first fixed pulley 2375, a second fixed pulley 2376, a guide element 2377, an elastic element 2378, and a rope 2379. The movable pulley 2374 is eccentrically mounted on the third rotation shaft 2371. The first fixed pulley 2375 is concentrically mounted on the third rotation shaft 2371, while the second fixed pulley 2376 is concentrically mounted on a fixed shaft (not signed in drawings) parallel to the third rotation shaft 2371. One end of the rope 2379 is fixed relative to the third rod 233, and the other end is wound around the movable pulley 2374, the first fixed pulley 2375, and the second fixed pulley 2376, and connected to one end of the elastic element 2379 via the guide element 2377 sequentially. The other end of the elastic element 2379 is fixed to the third rod 233.

More specifically, the third rod 233 comprises a first housing 2331 and a second housing 2332. The third rotation shaft 2371, the motor rotor 2372, the motor stator 2373, and the torque compensation mechanism are disposed within the space formed by the first housing 2331 and the second housing 2332 and located within a section of the third rod 233 that is closer to the third rotation joint 237. The fixed shaft, on which the second fixed pulley 2376 is mounted, is mounted above the third rotation shaft 2371 and within the section of the third rod 233 that is closer to the third rotation joint 237.

The distance from the axle center of the movable pulley 2374 to the axle center of the first fixed pulley 2375 is a constant value, that is, the eccentricity of the movable pulley 2374 relative to the third rotation axle center J3 is a constant value determined by the length of the mounting arm of the movable pulley 2374. The distance from the axle center of the second fixed pulley 2376 to the axle center of the first movable pulley 2375, i.e. the distance between the fixed shaft and the third rotation shaft 2371, is also a constant value. However, the distance from the axle center of the movable pulley 2374 to the axle center of the second fixed pulley 2376 varies with change of the angle of the movable pulley 2374 relative to the third rotation axle center J3. Since the movable pulley 2374 rotates with the third rotation shaft 2371, its angle relative to the third rotation axle center J3 is the rotation angle of the third rotation shaft 2371, i.e. the rotation angle of the second rod 232 relative to the third rod 233. In the illustrated embodiment, the rotating parts of the movable pulley 2374, the first fixed pulley 2375, and the second fixed pulley 2376 have equal diameters, facilitating the operation and parameter configuration of the torque compensation mechanism.

In the depicted embodiment, one end of the rope 2379 is fixed to the fixed shaft to which the second fixed pulley 2376 is mounted. More specifically, one end of the rope 2379 is fixed to the wire pulley on the fixed shaft, and the diameter of the wire pulley is equal to the diameter of the rope 2379 wound around the second fixed pulley 2376. To ensure the reliability of the rope 2379, a wear-resistant, length-invariant rope such as steel wire rope can be used as the rope 2379.

The guide element 2377 is fixed within the third rod 233 and disposed on a portion of the rope 2379 between the second fixed pulley 2376 and the elastic element 2378. The rope 2379 is wound around the guide element 2377 so that a portion of the rope between the guide element 2377 and the elastic element 2378 is in a vertical direction, which allows the elastic element 2378 to apply a vertical tensile force to one end of the rope 2378.

The elastic element 2378 provides tensile force to one end of the rope 2379. In the illustrated embodiment, the elastic element 2378 is a tension spring. One end of the tension spring is fixedly connected to the bottom of the third rod 233. The tension spring has advantages such as simple structure, long lifespan, lightness, and stable elastic force and can effectively balance the gravity torque of the second rod 232 without significantly increasing the structural complexity or overall weight of the third rotation joint 237. It can be understood that the torque compensation mechanism balances the gravity torque of the third rotation joint 237 of the master-hand wrist 23 by the elastic force of the elastic element. In other embodiments, materials such as rubber or elastic ropes can replace the tension spring. Additionally, in some embodiments, the elastic element 2378 can be replaced by a linear motor which pulls the rope 2379 to dynamically adjust torque balance.

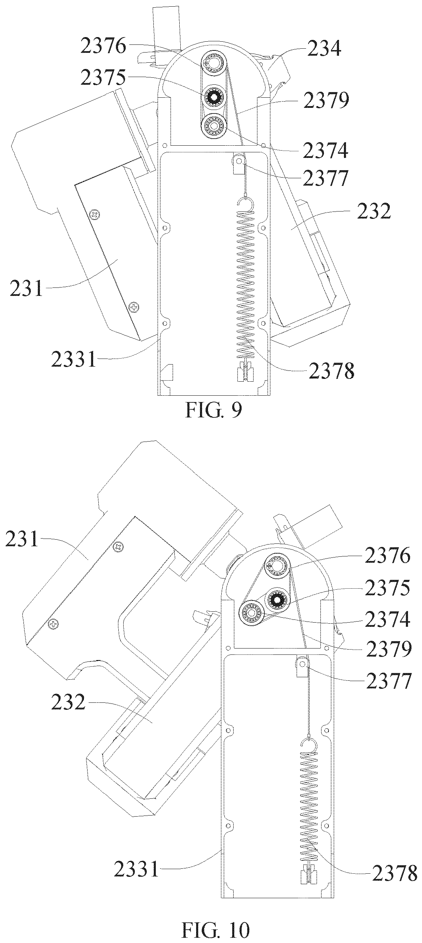

Please refer to FIGs. 9 to 11. Since the load carried by the third rotation shaft 2371 is a determined value, the initial tension (initial length) of the elastic element 2378 can be set based on the compensation torque required for the third rotation shaft 2371 at its initial position (zero position) to balance the gravity. The stretched length of the elastic element 2378 varies with the distance from the axle center of the movable pulley 2374 to the axle center of the second fixed pulley 2376, that is, the angle of the movable pulley 2374 relative to the third rotation axis J3 (as shown in different aspects in FIGs. 9 to 11). By establishing the relationship of change in rope length between the movable pulley 2374 and the second fixed pulley 2376 (i.e., the change of the stretched length of the elastic element) and the rotation angle of the third rotation shaft 2371 relative to the third rod 233, the stiffness coefficient K of the elastic element 2378 can be selected so that the compensation torque provided by the elastic element 2378 can be balanced with the torque on the third rotation shaft 2371 from the load.

Referring to Fig. 9, the movable pulley 2374 is located directly below the first movable pulley 2375. The first movable pulley 2375, the movable pulley 2374, and the second fixed pulley 2376 are aligned vertically, that is, the angle between the line connecting the axle center of the movable pulley 2374 and the third rotation axis J3 and to the line connecting the axle centers of the first fixed pulley 2375 and the second fixed pulley 2376 is 0° (zero position).

Referring to FIG. 10, the movable pulley 2374 rotates 30° clockwise about the third rotation axis J3 with respect to the zero position shown in FIG. 9. The movable pulley 2374 is located to the left and below the first movable pulley 2375, that is, the angle between the line connecting the axle center of the movable pulley 2374 and the third rotation axis J3 and to the line connecting the axle centers of the first fixed pulley 2375 and the second fixed pulley 2376 is 30° (in the clockwise direction).

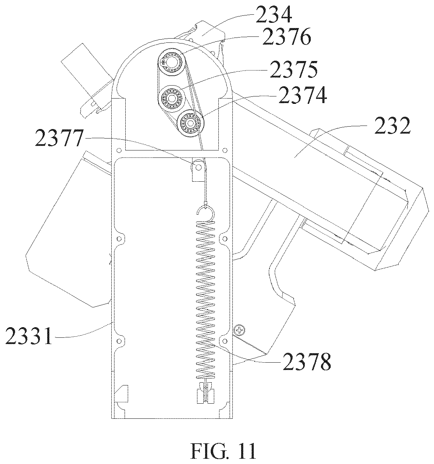

Referring to FIG. 11, the movable pulley 2374 rotates 30° counterclockwise about the third rotation axis J3 relative to the zero position shown in FIG. 9. The movable pulley 2374 is located to the right and slightly below the first movable pulley 2375, that is, the angle between the line connecting the axle center of the movable pulley 2374 and the third rotation axis J3 and to the line connecting the axle centers of the first fixed pulley 2375 and the second fixed pulley 2376 is 30° (in the counterclockwise direction).

In some other embodiments, a crank and a linkage can be used in the torque compensation mechanism as replacement of the movable pulley 2374, the first fixed pulley 2375, and the second fixed pulley 2376. That is, in the torque compensation mechanism, one end of the crank is mounted on the third rotation shaft 2371, and the other end of the crank is connected to the elastic element 2378 via a linkage. Therefore, the crank rotating with the third rotation shaft 2371, and the tension of the elastic element 2378 varies with the change of the position of the crank, thereby providing torque compensation.

It should be understood that the third rotation joint 237 of the master operating-hand 23 carries the maximum load, and thus the third rotation joint 237 is taken as an example. In other embodiments, torque compensation mechanism(s) can also be provided in at least one of the second rotation joint 236 in addition to the corresponding second driving element and the first rotation joint 235 in addition to the corresponding first driving element. Specifically, the first rotation joint 235 includes a first rotation shaft arranged along the first rotation axis J1 and a first driving element mounted on the first rotation shaft. One end of the first rotation shaft is fixedly connected to the handle 234, and the other end is rotatably connected to the first rod 231. The first driving element provides the power for the first rotation shaft to rotate about the first rotation axis. The second rotation joint 236 includes a second rotation shaft arranged along the second rotation axis J2 and a second driving element mounted on the second rotation shaft. One end of the second rotation shaft is fixedly connected to the first rod 231, and the other end is rotatably connected to the second rod 232. The second driving element provides the driving force for the second rotation shaft to rotate about the second rotation axis. The torque compensation mechanism(s) provided in at least one of the second rotation joint 236 and the first rotation joint 235 can have structure similar to that of the third rotation joint 237, which will not be further described herein.

The master-hand wrist, master operating device, and corresponding surgical robot provided by the present disclosure can utilize force (torque) compensation to achieve the effect of driving the rotation joint with driving element providing less driving force, which effectively reduces the inertia effect of the distal end, increases force transparency, enables easy operation even when the driving element is powered off, relieve or even prevent fatigue during the operation process for doctors, and ensures surgical effectiveness.

The technical features of the above-mentioned embodiments can be combined in any manner. To simplify the description, not all possible combinations of the technical features in the above-mentioned embodiments are described. However, as long as the combinations of these technical features do not contradict each other, they should all be considered as falling within the scope described in this specification.

The above-mentioned embodiments only express several implementation modes of the present disclosure, and the description is relatively specific and detailed, but they cannot be understood as limiting the scope of the patent application. It should be noted that for one skilled in the art, without departing from the concept of the present disclosure, several modifications and improvements can be made, and these all belong to the protection scope of the present disclosure. Therefore, the protection scope of the patent of the present disclosure should be subject to the appended claims.

## Claims

1. A master-hand wrist, comprising:
a handle;
a first rod rotatably connected to the handle about a first rotation axis via a first rotation joint;
a second rod rotatably connected to the first rod about a second rotation axis via a second rotation joint; and
a third rod rotatably connected to the second rod about a third rotation axis via a third rotation joint;
wherein the third rotation joint is provided with a torque compensation mechanism about the third rotation axis to compensate for torque generated by the handle, the first rod, and the second rod about the third rotation axis;
the third rotation joint comprises a third rotation shaft arranged along the third rotation axis, one end of the third rotation shaft is fixedly connected to the second rod, and the other end of the third rotation shaft is rotatably connected to the third rod; and
the torque compensation mechanism comprises a movable pulley eccentrically mounted on the third rotation shaft, a first fixed pulley concentrically mounted on the third rotation shaft, a second fixed pulley concentrically mounted on a fixed shaft parallel to the third rotation shaft, an elastic element, and a rope having one end fixed relative to the third rod and the other end wound around the movable pulley, the first fixed pulley, the second fixed pulley and connected to one end of the elastic element sequentially, and the other end of the elastic element is fixed on the third rod.

2. The master-hand wrist of claim 1, wherein the fixed shaft is mounted inside the third rod and above the third rotation shaft, and diameters of sliding portion of the movable pulley, the first fixed pulley, and the second fixed pulley are equal.

3. The master-hand wrist of claim 1, wherein the torque compensation mechanism comprises a guide element fixed on the third rod and disposed on a portion of the rope between the second fixed pulley and the elastic element, and the rope is wound around the guide element so that a portion of the rope between the guide element and the elastic element is in a vertical direction.

4. The master-hand wrist of claim 1, wherein the elastic element is a tensile elastic element, and an initial length of the elastic element is determined by achieving torque balance of the third rotation shaft at an initial position; a stiffness coefficient of the elastic element is determined based on a relationship between a rotation angle of the third rotation shaft relative to the third rod and magnitude of required compensation torque.

5. The master-hand wrist of claim 1, wherein a distance from an axle center of the movable pulley to an axle center of the second fixed pulley varies with change of an angle of the movable pulley relative to the third rotation axis.

6. A master-hand wrist, comprising:
a handle;
a first rod rotatably connected to the handle about a first rotation axis via a first rotation joint;
a second rod rotatably connected to the first rod about a second rotation axis via a second rotation joint; and
a third rod rotatably connected to the second rod about a third rotation axis via a third rotation joint;
wherein the third rotation joint is provided with a torque compensation mechanism about the third rotation axis to compensate for the torque generated by the handle, the first rod, and the second rod about the third rotation axis.

7. The master-hand wrist of claim 6, wherein the third rotation joint comprises a third rotation shaft arranged along the third rotation axis and a driving element mounted on the third rotation shaft, one end of the third rotation shaft is fixedly connected to the second rod, the other end of the third rotation shaft is rotatably connected to the third rod, and the driving element provides power for the third rotation shaft to rotate about the third rotation axis.

8. The master-hand wrist of claim 7, wherein the driving element comprises a motor rotor and a motor stator, the motor rotor is fixed on the third rotation shaft, and the motor stator is coaxially arranged outside the motor rotor.

9. The master-hand wrist of claim 7, wherein the torque compensation mechanism comprises a movable pulley eccentrically mounted on the third rotation shaft, a first fixed pulley concentrically mounted on the third rotation shaft, a second fixed pulley concentrically mounted on a fixed shaft parallel to the third rotation shaft, an elastic element, and a rope having one end fixed relative to the third rod and the other end wound around the movable pulley, the first fixed pulley, the second fixed pulley and connected to one end of the elastic element sequentially, and the other end of the elastic element is fixed on the third rod.

10. The master-hand wrist of claim 9, wherein the fixed shaft is mounted inside the third rod and above the third rotation shaft, and diameters of sliding portions of the movable pulley, the first fixed pulley, and the second fixed pulley are equal.

11. The master-hand wrist of claim 9, wherein the torque compensation mechanism comprises a guide element fixed on the third rod and disposed on a portion of the rope between the second fixed pulley and the elastic element, and the rope is wound around the guide element so that a portion of the rope between the guide element and the elastic element is in a vertical direction.

12. The master-hand wrist of claim 9, wherein the elastic element is a tensile elastic element, and an initial length of the elastic element is determined by achieving torque balance of the third rotation shaft at an initial position; a stiffness coefficient of the elastic element is determined based on a relationship between a rotation angle of the third rotation shaft relative to the third rod and magnitude of required compensation torque.

13. The master-hand wrist of claim 9, wherein a distance from an axle center of the movable pulley to an axle center of the second fixed pulley varies with change of an angle of the movable pulley relative to the third rotation axis.

14. The master-hand wrist of claim 7, wherein the torque compensation mechanism comprises a movable pulley eccentrically mounted on the third rotation shaft, a first fixed pulley concentrically mounted on the third rotation shaft, a second fixed pulley concentrically mounted on a fixed shaft parallel to the third rotation shaft, a linear motor, and a rope having one end fixed relative to the third rod and the other end wound around the movable pulley, the first fixed pulley, the second fixed pulley and connected to one end of the linear motor sequentially, and the other end of the linear motor is fixed on the third rod.

15. The master-hand wrist of claim 7, wherein the first rotation joint comprises a first rotation shaft arranged along the first rotation axis and a first driving element mounted on the first rotation shaft, one end of the first rotation shaft is fixedly connected to the handle, the other end of the first rotation shaft is rotatably connected to the first rod, and the first driving element provides power for the first rotation shaft to rotate about the first rotation axis; the second rotation joint comprises a second rotation shaft arranged along the second rotation axis and a second driving element mounted on the second rotation shaft, one end of the second rotation shaft is fixedly connected to the first rod, the other end of the second rotation shaft is rotatably connected to the second rod, and the second driving element provides power for the second rotation shaft to rotate about the second rotation axis.

16. The master-hand wrist of claim 15, wherein at least one of the first rotation joint and the second rotation joint is provided with a torque compensation mechanism.

17. The master-hand wrist of claim 6, wherein the first rotation axis, the second rotation axis, and the third rotation axis intersect at one point.

18. A master-hand wrist, comprising:
a handle;
a first rod rotatably connected to the handle about a first rotation axis via a first rotation joint;
a second rod rotatably connected to the first rod about a second rotation axis via a second rotation joint;
a third rod rotatably connected to the second rod about a third rotation axis via a third rotation joint; and
at least one torque compensation mechanism to compensate for torque generated by at least one of the handle, the first rod, the second rod, and the third rod about the first, the second, or the third rotation axis.

19. The master-hand wrist of claim 18, wherein the at least one torque compensation mechanism is one torque compensation mechanism connected to the third rod for compensating torque generated by the handle, the first rod, and the second rod about the third rotation axis.

20. The master-hand wrist of claim 18, wherein the at least one torque compensation mechanism are a plurality of torque compensation mechanisms.
